Europäisches Patentamt

European Patent Office

Office européen des brevets

(19)

(11) Veröffentlichungsnummer : **0 275 281 B1**

## (12) EUROPÄISCHE PATENTSCHRIFT

(45) Veröffentlichungstag der Patentschrift :
**09.10.91 Patentblatt 91/41**

(51) Int. Cl.⁵ : **H05G 1/30, H05G 1/46,
H05G 1/54, H05G 1/70**

(21) Anmeldenummer : **87904682.9**

(22) Anmeldetag : **23.07.87**

(86) Internationale Anmeldenummer :
**PCT/DE87/00332**

(87) Internationale Veröffentlichungsnummer :
**WO 88/00789 28.01.88 Gazette 88/03**

(54) **RÖNTGENANLAGE ZUR RÖNTGENDIAGNOSTIK UND/ODER RÖNTGENBEHANDLUNG.**

(30) Priorität : **23.07.86 DE 3624901**

(43) Veröffentlichungstag der Anmeldung :
**27.07.88 Patentblatt 88/30**

(45) Bekanntmachung des Hinweises auf die
Patenterteilung :
**09.10.91 Patentblatt 91/41**

(84) Benannte Vertragsstaaten :
**AT BE CH DE FR GB IT LI NL SE**

(56) Entgegenhaltungen :
**EP-A- 0 001 640
EP-A- 0 201 891
DE-A- 2 220 444
DE-A- 3 324 537
DE-A- 3 330 116
GB-A- 2 151 890
US-A- 3 932 759**

(73) Patentinhaber : **PICKER INTERNATIONAL
GMBH
Bärmannstrasse 38
W-8000 München 60 (DE)**

(72) Erfinder : **EILS, Friedrich
Hauptstr. 96
W-2841 Wagenfeld 1 (DE)**
Erfinder : **MÜLLER, Wolfgang
Graudenzer Weg 10
W-4992 Espelkamp (DE)**
Erfinder : **SCHNEIDER, Eli, J.
Wollriede 2
W-4992 Espelkamp (DE)**
Erfinder : **TAUBITZ, Peter
Leipzigerstr. 58
W-4992 Espelkamp (DE)**
Erfinder : **KNIPPING, Ernst
Lessingstr. 9
W-4990 Lübbecke (DE)**

(74) Vertreter : **Patentanwälte Viering & Jentschura
Steinsdorfstrasse 6 Postfach 22 14 43
W-8000 München 22 (DE)**

**Beschreibung**

Die Erfindung betrifft eine Röntgenanlage zur Röntgendiagnostik und/oder Röntgenbehandlung, mit einem Bedienpult mit Funktionstasten zum Abrufen organprogrammierter Einstellwerte für einen Röntgengenerator aus einem Programmspeicher und einer vielstelligen alphanumerischen Anzeigeeinrichtung am Bedienpult zum Anzeigen von aus dem Programmspeicher abgerufenen Einstellwerten.

Bei einer bekannten Röntgendiagnostikanlage (DE-AS 23 11 211) sind an einem Bedienpult Funktionstasten zum Abrufen organprogrammierter Einstellwerte für einen Röntgengenerator und ein angeschlossenes Röntgenuntersuchungsgerät aus einem Programmspeicher angeordnet. Die von der jeweiligen Funktionstaste abgerufenen Einstellwerte sind in einer dieser Funktionstaste zugeordneten Speicherzelle des Programmspeichers abgelegt und werden durch Betätigen der Funktionstaste an einer Anzeigeeinrichtung am Bedienpult angezeigt. Die Anzeigeeinrichtung enthält numerische Anzeigefelder zum Anzeigen der Röntgenröhrenspannung und des mAs-Produktes, sowie Anzeigelampen zum Anzeigen jeweils eingestellter Zusatzfunktionen. Es ist auch möglich, die Einstellwerte für den Röntgengenerator und das Röntgenuntersuchungsgerät über den Anzeigegliedern der Anzeigeeinrichtung zugeordnete Einstellglieder frei zu wählen sowie die gewählten Einstellwerte durch Betätigen eines Programmierschalters und einer der Funktionstasten in die jeweils zugeordnete Speicherzelle des Programmspeichers einzuspeichern.

Hierbei ist es jedoch erforderlich, die Funktionstasten organbezogen zu kennzeichnen, was durch Beschriftung eines an der jeweiligen Funktionstaste vorhandenen Beschriftungsstreifens mit einem entsprechenden "Organtext" erfolgen kann, der jedoch auf solchen Beschriftungsstreifen aus Platzgründen nur in stark abgekürzter Form angegeben werden kann. Dies kann durch Betätigen einer falschen Funktionstaste z.B. zur Einstellung von für den jeweiligen Untersuchungsfall ungeeigneten oder unzulässigen Einstellwerten führen, die ihrerseits an der Anzeigevorrichtung lediglich ihrer Größe nach und teilweise in über Anzeigelampen verschlüsselter Form angezeigt werden und dadurch selbst keinen deutlichen Hinweis darauf liefern, auf welche Organaufnahme sie bezogen sind.

Bei bekannten Röntgenanlagen vorliegender Art kann ggf. auch durch Anzeigelampen angezeigt werden, falls ein Bedienungs- oder Gerätefehler aufgetreten ist oder der Röntgengenerator oder das angeschlossene Röntgenaufnahmegerät für eine Aufnahme unter den eingestellten Aufnahmebedingungen nicht bereit ist. Hierbei kann jedoch allenfalls anhand von Fehlerlisten oder der Bedienungsanleitung und dgl. festgestellt werden, was die Ursache für die Nichtbereitschaft der Anlage ist oder was für ein Fehler aufgetreten ist, damit die Bedienungsperson ggf. entsprechend eingreifen kann.

Aus der EP-A-0 001 640 ist eine radiologische Einrichtung zur Diagnostik bekannt, wobei der Abruf von Belichtungsfaktoren auf einer anatomischen Basis durch Betätigen eines Satzes von Druckknöpfen, die z.B. den anatomischen Bereich, die Technik, den gewünschten Blickwinkel, die Patientendicke u.s.w. spezifizieren, erfolgen kann. Der Rechner dieser Einrichtung ist mit Anzeige- und Ausgabeeinheiten gekoppelt, die unter anderem alphanumerische Anzeigen aufweisen können und für die Informationsübermittlung an den Benutzer oder das Hilfspersonal über die jeweilige Systemoperation geeignet sind. Es ist jedoch nicht konkretisiert, um welche Art von Informationsübermittlung es sich hierbei handelt.

Durch die Erfindung wird die Aufgabe gelöst, eine Röntgenanlage der eingangs erwähnten Art zu schaffen und derart auszubilden, daß leicht verständliche und hinreichend ausführliche Informationen zur Erleichterung der Bedienung geliefert werden können.

Dies wird erfindungsgemäß dadurch erreicht, daß der Programmspeicher einen Organtext-Speicherteil mit mehreren den Funktionstasten jeweils zugeordneten Organtext-Speicherplätzen enthält, deren Inhalt durch das Betätigen der jeweils zugeordneten Funktionstasten in die Anzeigeeinrichtung, d.i. in die Anzeigefelder, abrufbar ist, und daß der Programmspeicher und die Anzeigeeinrichtung, über eine Schalteranordnung an einen Vorratsspeicher für Organtexte und zugeordnete Einstellwerte anschließbar ist, aus welchem ein jeweiliger Organtext und die zugeordneten Einstellwerte durch Betätigen eines Abrufschalters in die Anzeigeeinrichtung, und durch Betätigen einer Funktionstaste in den dieser zugeordneten Speicherplatz des Programmspeichers abrufbar sind.

Durch den erfindungsgemäßen Vorschlag, den Programmspeicher mit einem Organtext-Speicherteil zu versehen, werden die Organtexte durch Betätigen einer Funktionstaste zusammen mit den zugeordneten Einstellwerten in der alphanumerischen Anzeigeeinrichtung unverschlüsselt im Klarworttext - auch mit längeren und ggf. mehreren Worten - (z.B. "Schädelübersicht a.p./p.a.", "Schädel seitlich", "Schädel a.p.", "Kniegelenk", "Kniegelenk nach Frick") angegeben. Weiterhin kann, da der Programmspeicher mit Hilfe des Vorratsspeichers in der beschriebenen Weise programmierbar ist, das Bedinungspersonal die Zuordnung der Funktionstasten zu den Einstellwerten und den Organtexten nach Bedarf selbstständig durchführen.

Es wird bevorzugt, wenn die vielstellige alphanumerische Anzeigeeinrichtung zwei getrennte Anzeigefelder aufweist und die Einstellwerte in das erste Anzeigefeld und die Organtexte in das zweite Anzeigefeld abrufbar sind.

Weiterhin ist es auch möglich, für den Vorratsspeicher einen Tastaturanschluß zur Programmierung des Vorratsspeichers vorzusehen. Es besteht auch die Möglichkeit, einen Tastaturanschluß für die alphanumerische Anzeigevorrichtung vorzusehen. In beiden Fällen kann von der Bedienungsperson über den Tastaturanschluß derjenige Organtext über den Vorratsspeicher oder die Anzeigeeinrichtung in den Programmspeicher eingelesen werden, der ihr zur Beschreibung der Organzuordnung zu der jeweiligen Funktionstaste am schnellsten und deutlichsten geeignet erscheint. Falls der die Organtexte anzeigende Teil der Anzeigevorrichtung oder der die Organtexte speichernde Teil des Vorratsspeichers von deren die Einstellwerte anzeigenden bzw. speichernden Teil über den Tastaturanschluß unabhänging ansteuerbar ist, lassen sich mittels der Tastatur den gespeicherten und angezeigten Einstellwerten ohne deren Änderung willkürlich formulierte Organtexte einlesen und zuordnen.

Die erfindungsgemäße Röntgenanlage kann weiterhin ein elektronisches Überwachungssystem zur Überwachung der Anlage auf Funktionsbereitschaft, Bedienungs- und Gerätefehler, Überschreitung bzw. Unterschreitung zulässiger Grenzwerte und dgl. aufweisen Hierbei ist Überwachungssystem ein Überwachungst extspeicher zugeordnet, an welchen die vielstellige alphanumerische Anzeigeeinrichtung von dem Überwachungssystem programmgesteuert anschließbar ist. Bei einer solchen Ausführungsform der Erfindung können von der Anzeigeeinrichtung entsprechende Informationen über Art und ggf. Ursache mangelnder Funktionszustände der Anlage, wie z.B. "Übersteuerung" bei Vorliegen einer zu hohen Röntgenröhrenspannung oder "Bedienungsfehler" wörtlich im Klartext angezeigt werden. Es ist auch möglich, Maßnahmen zur Fehlerbeseitigung im Klartext anzuzeigen, z.B. "Tür schließen", "Überstromschalter einschalten", "Generator aus/einschalten", was über entsprechende Schalter und die Programmsteuerung auch im Dialogverkehr erfolgen kann. Ein Nachlesen z.B. in Fehlerlisten und der Bedienungsanleitung kann dadurch überflüssig werden. Das Anzeigen solcher Überwachungstexte kann so erfolgen, daß andere angezeigte Daten von ihnen "überblendet" werden. Falls im aktuellen Fall erforderlich, wird außerdem die Auslösung der Röntgenaufnahme von der Überwachungseinrichtung verhindert, bis der Fehler beseitigt ist.

Bevorzugt ist außerdem der Textspeicher des Überwachungssystems an einen Fehlertext-Ablagespeicher angeschlossen, in welchem ein Fehler oder dgl. durch dessen Auftreten selbsttätig dokumentiert wird und welcher über eine Schalteranordnung und einen Abrufschalter an die Anzeigeeinrichtung anschließbar ist. Hierdurch wird dem Wartungs- und Reparaturtechniker die Geschichte der aufgetretenen Fehler im Klarworttext angezeigt.

In weiterer Ausgestaltung der Erfindung kann die Anzeigeeinrichtung über eine Schalteranordnung an einen Textspeicher eines elektronischen Informationssystems anschließbar sein, aus dem über einen Abrufschalter bestimmte Informationen, wie nicht ständig angezeigte Betriebsparameter, ggf. programmgesteuert per Dialog, in der Anzeigeeinrichtung im Klartext angezeigt werden können, z.B. Phototimerangaben, Folientypangaben, Aufnahmezählerstände, Betriebsstunden, Datum, Uhrzeit und dgl.

Bei einer weiteren bevorzugten Ausgestaltung der Erfindung sind die Anzeigeeinrichtung und ein Tastaturanschluß ggf. nach Betätigen eines zugeordneten Schalters an einen Druckerspeicher angeschlossen, der über einen Abrufschalter in einen Drucker, insbesondere einen Etikettendrucker, auslesbar ist. Dadurch können nach Aufnahmeende alle Aufnahmedaten und die vorher eingegebenen Personendaten mittels des Druckers aufgezeichnet werden, was bisher handschriftlich erfolgen mußte.

Vorzugsweise kann die vielstellige alphanumerische Anzeigeeinrichtung über eine Schalteranordnung und einen Auswählschalter an einen Prüftextspeicher für eine in die Röntgenanlage integrierte programmierte Prüfelektronik anschließbar sein. Dadurch können programmgesteuert elektrische Module und Baugruppen, Signalgeber und dgl. abgerufen und über den zugeordneten Prüftextspeicher und mit Hilfe der Klarworttext-Anzeigeeinrichtung Einzel- bzw. Funktionstest von z.B. Speichern, Signal-Ein- und Ausgängen, Baugruppen und Funktionen des Gesamtsystems im Dialogverkehr durchgeführt werden. Durch den Dialog und ggf. das Anzeigen von Fehlerbeseitigungshinweisen im Klarworttext werden dem Wartungstechniker Fehleranalysen und die Fehlerbeseitigung erleichtert. Auf das Anschließen externer Test- und Bediengeräte kann daher weitestgehend verzichtet werden.

Bei einer weiteren Ausführungsfom der Erfindung sind mehrere Röntgengeräte, die von einem gemeinsamen Bedienpult aus durch Betätigen eines zugeordneten Schalters an den Röntgengenerator und die erforderlichen Funktionsgruppen angeschaltet werden können, über eine Schaltverknüpfungsmatrix angeschlossen. Diese Schaltverknüpfungsmatrix ist über eine Schalteranordnung durch Betätigen von Steuerschaltern programmgesteuert umschaltbar. Weiter ist der Schaltverknüpfungsmatrix ein Schaltverknüpfungstextspeicher zugeordnet, dessen Speicherplätze über die Schalteranordnung durch Betätigen der Steuerschalter in die vielstellige alphanumerische Anzeigeeinrichtung programmgesteuert und im Dialogverkehr in Abhängigkeit von der jeweils durchgeführten Schaltverknüpfung abgerufen werden können, so daß von der Anzeigeeinrichtung entsprechende Klarworttexthinweise angezeigt werden.

Bei bisher bekannten Röntgenanlagen konnten Geräteanpassungen ggf. nur durch Umstecken von Steckbrücken oder Anlöten von Signalleitungen und dgl. durchgeführt werden. Durch die erfindungsgemäß vorgesehene, programmierbare Schaltverknüpfungsmatrix hingegen ist es mit Hilfe der alphanumerischen Anzeigeeinrichtung möglich, Änderungen der Schaltverknüpfungen im Dialogverkehr aufgrund von angezeigten Klartextinformationen durchzuführen.

Gemäß der Erfindung sind also in einer Röntgenanlage zur Röntgendiagnostik und/oder Rötgenbehandlung ein Textspeicher und an einem Bedienpult der Röntgenanlage eine vielstellige alphanumerische ein- oder mehrzeilige Anzeigeeinrichtung vorgesehen, in welche in dem Textspeicher einprogrammierte Informationen selbsttätig oder durch Betätigen von Schaltern, insbesondere der Bedientasten des Bedienpultes, abgerufen werden können und in der Anzeigeeinrichtung unverschlüsselt im Klarworttext auch mit längeren und ggf. mehreren Worten angegeben werden können.

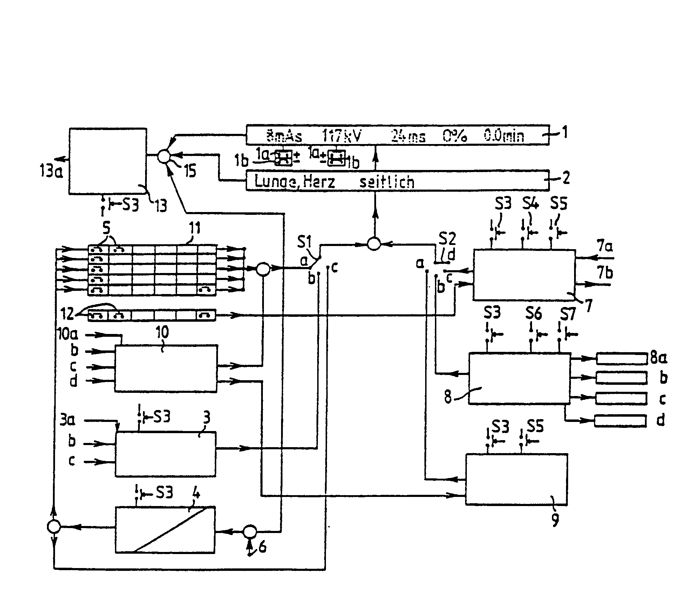

Die Erfindung wird anhand eines gegenwärtig bevorzugten Ausführungsbeispiels anhand der Zeichnung erläutert. Hierbei sind im wesentlichen nur solche Bestandteile for Röntgenanlage gezeigt, von denen die alphanumerische Anzeigevorrichtung an dem Bedienpult der Anzeigevorrichtung angesteuert werden kann.

Die erfindungsgemäß im Bedienteil eines Röntgengengenerators integrierte vielstellige alphanumerische Anzeigeeinrichtung weist im Ausführungsbeispiel zwei jeweils einzeilige Anzeigefelder 1 und 2 auf, in denen im Zusammenwirken mit Textspeichern unter einer Programmsteuerung durch Betätigen von Schaltern auch längere Hinweise und Informationen im Klartext angezeigt werden können. Im gezeigten Ausführungsbeispiel können von dem Anzeigefeld 1 alle einstellbaren Einstellwerte des Röntgengenerators für eine durchzuführende Röntgenaufnahme, wie Röntenröhrenspannung, mAs-Produkte, Belichtungszeit und dgl. angezeigt werden. In der Zeichnung sind als Beispiel Tasten 1a und 1b gezeigt, mit welchen solche Einstellwerte auch manuell durch Aufsteuern oder Absteuern verändert werden können. Das Anzeigefeld 2 hingegen ist zum Anzeigen von Worttextinformationen vorgesehen, die ggf. im Dialogverkehr aus Speicherplätzen je nach der Stellung von Auswählschaltern S1 und S2 zugeschalteter Textspeicher abgerufen werden können.

Dadurch liefert die alphanumerische Anzeigeeinrichtung in der gezeigten Stellung S1-a des Schalters S1 Klartextanzeigen für Bedienerführung, vorgenommene und durchzuführende Einstellungen, Organtexte, Grenzwerte, vorliegende Bedien- und Systemfehler und dgl. mehr, wodurch der Bedienungsperson und dem Wartungstechniker das Lesen einer Bedienungsanleitung weitestgehend erspart bleibt.

Außer den vorstehend erwähnten Klartextanzeigen ergeben sich für die Bedienungsperson durch Umschalten des Schalters S1 weitere Klartextinformationen, die in die Anzeigeeinrichtung 1, 2 abgerufen werden können. Durch Anwählen des integrierten Informationssystems 3 können alle ständig nicht angezeigten Betriebsparameter, wie z.B. Zählerstände der Aufnahmezähler, Angaben zu den Aufnahmefolien, Betriebsstunden, Datum, Uhrzeit und dgl. im Klartext in die Anzeige felder 1, 2 abgerufen werden. Durch Anwählen eines Organtext-Vorratsspeichers 4 besteht die Möpglichkeit, jeder Funktionstaste 5 über einen Programmspeicher 11 einen beliebigen Organtext zuzuordnen bzw. über eine Tastaturschnittstelle 6 neue Organtexte einzulesen.

Der Wartungstechniker hat außerdem durch Umschalten des Schalters S2 die Möglichkeit, über eine Verknüpfungsmatrix 7 Systemparameter-Anpassungen und Signalzuweisungen für Peripheriegeräte, insbesondere vom Bedienpult aus anwählbare Aufnahmegeräte, vorzunehmen, sowie ein integriertes elektronisches Prüfdiagnosesystem 8 zum Prüfen elektronischer Module, von Signalen oder von Baugruppen, und vorprogrammierte Testprogramme abzurufen oder durch Ansteuern des Fehlertext-Ablagespeichers die Fehlergeschichte abzufragen. Auch hierbei werden die Bedienerführungsangaben und alle anderen Angaben im Klarworttext angezeigt.

Im einzelnen sind in der gezeigten Grund-Schaltsellung S1-a des Schalters S1 ein Programmspeicher 11 zum Speichern organprogrammierter Einstellwerte mit zugeordneten Organtexten, die durch Betätigen einer zugeordneten Funktionstaste 5 abrufbar sind, sowie der Textspeicher eines integrierten elektronischen Überwachungssystems 10 angewählt. Unter der Steuerung des elektronischen Überwachungssystems werden über dessen Eingangsleitungen 10a bis 10d z.B. Bedienfehler, Fehler, Grenzwerte und Generatorfunktionen registriert, die durch selbsttätiges Abrufen zugeordneter Überwachungstexte aus einem Überwachungstextspeicher jeweils im Klarworttext in dem Anzeigefeld 2 der Anzeigeeinrichtung angezeigt werden.

In der Stellung S1-b des Schalters S1 ist das elektronische Informationssystem 3 angewählt. Durch Betätigen der Abruftaste S3 können alle nicht ständig angezeigten Betriebsparameter, programmgesteuert per Dialog, über die Eingangsleitungen 3a bis 3c abgerufen werden, z.B. Phototimerangaben, Folientypen, Aufnahmezählerstände, Betriebsstunden, Datum, Uhrzeit und dgl., und in dem Anzeigefeld 2 im Klartext wiedergegeben werden.

In der Schaltstellung S1-c des Schalters S1 ist der Organtext-Vorratsspeicher 4 angewählt. Mit der zugehörigen Auswahltaste S3 kann aus dem Organtextvorratsspeicher jeder Funktionstaste jeder Organtext (Klarworttext) zugewiesen werden, oder es kann über den Tastaturanschluß ein neuer Organtext eingelesen werden. Über eine zusätzliche Schnittstelle 15 können alle Aufnahmedaten und die vorher über den Tastaturanschluß 6 eingegebenen Personendaten in einen Druckerspeicher 13 eingelesen, und nach Aufnahmeende über den Ausgang 13a an einen Etikettendrucker oder dgl. ausgegeben werden.

Vom Wartungs- und Installationstechniker können zusätzlich zu den Bedienungsangaben weitere Informationen bzw. Einstellungen unter Bedienerführung per Klartext-Dialog durch Umschalten des Tasters S2 vorgenommen werden. In der Schalterstellung S2-a des Schalters S2 ist der Fehlertext-Ablagerspeicher 9 angewählt, aus dem durch Betätigen der Lesetaste S3, die über das Überwachungssystem 10 und dessen Überwachungstextspeicher bei Auftreten des jeweiligen Fehlers selbsttätig abgelegten Fehlertexe in die Anzeigefelder 1, 2 abgerufen und dort im Klarworttext angezeigt werden. In dieser Weise kann der Techniker über die vorhandene Fehlergeschichte im Klarworttext informiert werden. Die gespeicherten Fehlertexte können ggf. durch Betätigen der Löschtaste S5 wieder gelöscht werden.

In der Schalterstellung S2-b des Schalters S2 ist das in die Anlage integrierte Prüf- und Diagnosesystem 8 angewählt. Durch Betätigen des Auswähltasters S3 können im Klartextdialog, der an der Anzeigeeinrichtung 1, 2 angezeigt wird, entsprechende Einzel- bzw. Funktionstests von z.B. Speicher, Signal-Ein-Ausgängen, Baugruppen oder Funktionen 8a bis 8d des Gesamtsystems programmgesteuert ausgewählt werden. Über die Tastschalter S6 und S7 kann dann der entsprechende Text programmgesteuert gestartet bzw. gestoppt werden. Zustandsinformationen, ggf. Fehlerbehebungshinweise, werden im Klarworttext in der Anzeigeeinrichtung 1, 2 angezeigt.

In der Schalterstellung S2-c des Schalters S2 ist die Verknüpfungsmatrix angewählt, um gewünschte Schaltungsverknüpfungen zwischen den Eingängen 7a und Ausgängen 7b insbesondere mit den Röntgengerät-Auswähltastern 12 herzustellen oder zu lösen. Mittels des zugeordneten Auswähltasters S3 kann eine einzustellende Schaltungsverknüpfung im Dialogverkehr mit der Anzeigeeinrichtung 1, 2 ausgewählt und mit den Schalttastern S4 und S5 gesetzt bzw. gelöscht werden. Einstellungen sind hierbei z.B. Grenzwerte, Röhreneinstellungen und Zuweisungen von Eingangs- und Ausgangssignalen zum Anschluß externer Geräte an den Röntgengenerator. Ein Umstecken oder Umlöten von Signalleitungen für externe Geräte ist nicht mehr erforderlich, da über die programmierbare Verknüpfungsmatrix diese Signalzuweisungen im Klartextdialogverkehr mit der Anzeigeeinrichtung 1, 2 programmgesteuert vorgenommen werden können.

In der Schalterstellung S2-d des Schalters S2 sind die Techniker-Systeme 7 bis 9 ausgeschaltet.

Die angesprochenen Speicher können Einzelspeicher oder Speicherteile eines oder mehrerer gemeinsamer Speicher sein. Die im Ausführungsbeispiel als Drehschalter dargestellten Schalter S1 und S2 können durch einen einzigen Betätigungsschalter ersetzt werden. Es ist auch möglich, die entsprechenden Schaltstellungen durch Betätigungskombinationen sonstiger Bedienschalter des Bedienpultes zu erreichen.

## Patentansprüche

1. Röntgenanlage zur Röntgendiagnostik und-/oder Röntgenbehandlung, mit einem Bedienpult mit Funktionstasten zum Abrufen organprogrammierter Einstellwerte für einen Röntgengenerator und ein angeschlossenes Röntgengerät aus einem Programmspeicher und einer vielstelligen alphanumerischen Anzeigeeinrichtung am Bedienpult zum Anzeigen von aus dem Programmspeicher abgerufenen Einstellwerten, dadurch gekennzeichnet, daß der Programmspeicher (11) einen Organtext-Speicherteil mit mehreren den Funktionstasten jeweils zugeordneten Organtext-Speicherplätzen enthält, deren Inhalt durch das Betätigen der jeweils zugeordneten Funktionstasten (5) in die Anzeigeeinrichtung (1, 2) abrufbar ist, und daß der Programmspeicher (11) und die Anzeigeeinrichtung (1, 2) über eine Schalteranordnung (S1-c) an einen Vorratsspeicher (4) für Organtexte und zugeordnete Einstellwerte anschließbar ist, aus welchem ein jeweiliger Organtext und die zugeordneten Einstellwerte durch Betätigen eines Abrufschalters (4-S3) in die Anzeigeeinrichtung (Anzeigefelder 1, 2), und durch Betätigen einer Funktionstaste (5) in den dieser zugeordneten Speicherplatz des Programmspeichers (11) abrufbar sind.

2. Röntgenanlage nach Anspruch 1, dadurch gekennzeichnet, daß die vielstellige alphanumerische Anzeigeeinrichtung zwei getrennte Anzeigefelder (1, 2) aufweist und die Einstellwerte in das erste Anzeigefeld (1) und die Organtexte in das zweite Anzeigefeld (2) abrufbar sind.

3. Röntgenanlage nach einem der Ansprüche 1 oder 2, dadurch gekennzeichnet, daß dem ersten Anzeigefeld (1) Tasten (1a, 1b) zugeordnet sind, mit welchen die von dem ersten Anzeigefeld angezeigten Einstellwerte manuell durch Aufsteuern oder Absteuern verändert werden können.

4. Röntgenanlage nach einem der Ansprüche 1 bis 3, dadurch gekennzeichnet, daß der Vorratsspeicher (4) über einen Tastaturanschluß (6) programmierbar ist.

5. Röntgenanlage nach einem der Ansprüche 1 bis 4, gekennzeichnet durch ein elektronisches Überwachungssystem mit einem Überwachungstextspeicher an welchen die vielstellige alphanumerische Anzeigeeinrichtung (1, 2) zum Anzeigen von durch das Überwachungssystem abgefühlten Bedienfehlern, Gerätefehlern, unzulässigen Einstellwerten, überschrittenen Grenzwerten und dgl. von dem Überwachungssystem (10) gesteuert anschließbar ist.

6. Röntgenanlage nach Anspruch 5, dadurch gekennzeichnet, daß das Überwachungssystem (10) an einen Fehlertext-Ablagespeicher (9) angeschlossen ist, der über eine Schalteranordnung (S2-a) und einen Abrufschalter (9-S3) an die Anzeigeeinrichtung (1, 2) anschließbar ist.

7. Röntgenanlage nach einem der Ansprüche 1 bis 6, dadurch gekennzeichnet, daß die Anzeigeeinrichtung (1, 2) über eine Schalteranordnung (S1-B) an einen Textspeicher eines elektronischen Informationssystems (3) zum Anzeigen von mittels eines Abrufschalters (3-S3) aus dem Textspeicher in die Anzeigeeinrichtung (1, 2) abrufbaren Informationen anschließar ist.

8. Röntgenanlage nach einem der Ansprüche 1 bis 7, dadurch gekennzeichnet, daß die Anzeigeeinrichtung (1, 2) und ein Tastaturanschluß (6) an einen Druckerspeicher (13) angeschlossen sind, der über einen Abrufschalter (13-S3) in einen Drucker, insbesondere einen Etikettendrucker, auslesbar ist.

9. Röntgenanlage nach einem der Ansprüche 1 bis 8, gekennzeichnet durch eine integrierte programmierte Prüfelektronik (8) mit einem Prüftextspeicher, der über eine Schalteranordnung (S2-b) und einen Auswählschalter (8-S3) an die vielstellige alphanumerische Anzeigeeinrichtung (1, 2) anschließar ist.

10. Röntgenanlage nach einem der Ansprüche 1 bis 9, gekennzeichnet durch einen Röntgengenerator und mehrere von dem Bedienpult mit der Anzeigeeinrichtung aus durch Betätigen eines jeweils zugeordneten Schalters wahlweise anschaltbaren Röntgengeräten, welche über eine Schaltverknüpfungsmatrix (7) angeschlossen sind, die über eine Schalteranordnung (S2-c) durch Betätigen von Steuerschaltern (7-S3, 7-S4, 7-S5) programmgesteuert umschaltbar ist und der ein Schaltverknüpfungstextspeicher zugeordnet ist, welcher über die Schalteranordnung (S2-c) durch Betätigen der Steuerschalter in die vielstellige alphanumerische Anzeigeeinrichtung (1, 2) abrufbar ist.

## Claims

1. X-ray equipment for X-ray diagnostic and/or X-ray therapy comprising a control desk with function keys for recalling organ-programmed adjustment values for an X-ray generator and a connected X-ray apparatus from a program store and a multidigit alphanumeric display device on the control desk for displaying adjustment values recalled from the program store, characterized in that the program store (11) comprises an organ-text store unit with a plurality of organ-text storage locations respectively allocated to the function keys, wherein the content of said storage locations can be recalled to the display device (1, 2) by operating the respectively attributed function keys (5) and in that the program store (11) and the display device (1, 2) can be connected to a repertory store (4) for organ characterizing texts and attributed adjustment values through a switch device (S1-c), from this repertory store, a respective organ characterizing text and the attributed adjustment values can be recalled to the display device (displays 1, 2) by operating a recall switch (4-S3) and to a storage location of the program store (11) allocated to a function key (5) by operating the latter.

2. X-ray equipment according to claim 1, characterized in that the multidigit alphanumeric display device shows two separate displays (1, 2) and that the adjustment values can be recalled to the first display (1) and the organ characterizing texts to the second display (2).

3. X-ray equipment according to claim 1 or claim 2, characterized in that the first display is attributed keys (1a, 1b), by which the adjustment values displayed by the first display can be varied manually by controlling them up or down.

4. X-ray equipment according to any of the claims 1 to 3, characterized in that the repertory store (4) can be programmed by a key connection (6).

5. X-ray equipment according to any of the claims 1 to 4, characterized by an electronic guard system with a guard text store, to which the multidigit alphanumeric display device (1, 2) for displaying operating errors, equipment defects, forbidden adjustment values, exceeded boundary values and the like, sensored by the guard system, can be connected under the controll of the guard system (10).

6. X-ray equipment according to claim 5, characterized in that the guard system (10) is connected to a erroneous text bin-store (9) which can be connected to the display device (1,2) through a switch device (S2-a) and a recall switch (9-S3).

7. X-ray equipment according to any of the claims 1 to 6, characterized in that ,through a switch device, the display device (1, 2) can be connected to a text store of an electronic information system (3) for displaying informations which can be recalled from the text store to the display device by means of a recall switch (3-S3).

8. X-ray equipment according to any of the claims 1 to 7, characterized in that the display device (1,2) and a key connection (6) are connected to a printing store (13) which can be red-out to a printer, particularly a label printer, by means of a recall switch (13-S3).

9. X-ray equipment according to any of the claims 1 to 8, characterized by an integrated programmed control electronics (8) with a control text store which can be connected to the multidigit alphanumeric display device (1, 2) through a switch device (S2-b) and a select switch (8-S3).

10. X-ray equipment according to any of the claims 1 to 9, caracterized by an X-ray generator and a plurality of X-ray apparatus which can be selectedly switched on by operating a respectivly attributed switch on the control desk with the display device, the X-ray apparatus being connected through a connection matrix (7) which can be switched program-controlledly through a switch device (S2-c) by operating control keys (7-S3, 7-S4, 7-S5) and which is attributed a connection text store which can be recalled to the multidigit alphanumeric display store (1, 2) through the switch device (S2-c) by operating the function keys.

## Revendications

1. Installation radiographique pour diagnostic et/ou pour traitement radiographique, contenant un pupitre de commande avec des touches de fonction pour appeler d'une mémoire de programmes des valeurs de consigne programmées selon des organes pour un générateur radiographique et un appareil radiographique et en un dispositif d'affichage alphanumérique à plusieurs chiffres se trouvant au pupitre de commande pour indiquer des valeurs de consigne appelées de la mémoire de programmes, caractérisé en ce que la mémoire de programmes (11) contient une unité-mémoire de textes pour organes qui contient plusieurs emplacements de mémoire de textes pour organes respectivement coordonnées aux touches de fonction, le contenu desquelles peut être appelé sur le dispositif d'affichage (1, 2) en actionnant les touches de fonction (5) respectivement coordonnées, et que la mémoire de programmes (11) et le dispositif d'affichage (1, 2) soient branchables par un placement de commutateurs (S1-c) à une mémoire d'alimentation (4) pour des textes pour organes et pour des valeurs de consigne coordonnées, de laquelle un texte pour organes respectif et les valeurs de consigne coordonnées peuvent être appelés en actionnant un commutateur d'appel (4-S3) sur le dispositif d'affichage (1, 2) et en actionnant une touche de fonction (5) sur l'emplacement de mémoire de la mémoire de programmes (11) coordonné respectivement à cette touche de fonction.

2. Installation radiographique selon la revendication 1, caractérisé en ce que le dispositif d'affichage alphanumérique à plusieurs chiffres montre deux panneaux d'affichage (1, 2) séparés et les valeurs de consigne puissent être appelées sur le premier panneau d'affichage (1) et les textes pour organes sur le deuxième panneau d'affichage (2).

3. Installation radiographique selon l'une des revendications 1 ou 2, caractérisé en ce que des touches (1a, 1b) sont coordonnées au premier panneau d'affichage (1) avec lesquelles les valeurs de consigne indiquées par le premier panneau d'affichage peuvent être variées en les manoevrant de facon manuelle en haut ou en bas.

4. Installation radiographique selon l'une des revendications 1 à 3, caractérisé en ce que la mémoire d'alimentation (4) est programmable par un raccordement de clavier (6).

5. Installation radiographique selon l'une des revendications 1 à 4, caractérisé en ce qu'un système de surveillance électronique ayant une mémoire de textes de surveillance à laquelle le dispositif d'affichage alphanumérique à plusieurs chiffres (1, 2) soit branchable et destiné à l'indication des erreurs de manipulation, des défaillances machine, des valeurs de consigne inacceptables, des valeurs limites dépassées et des autres choses semblables explorées par le système de surveillance (10) et reglées par celle-ci.

6. Installation radiographique selon la revendication 5, caractérisé en ce que le système de surveillance (10) soit branché à une mémoire de dépot pour textes des erreurs (9) qui est branchable par un placement de commutateurs (S2-a) et par un commutateur d'appel (9-S3) au dispositif d'affichage (1, 2).

7. Installation radiographique selon l'une des revendications 1 à 6, caractérisé en ce que le dispositif d'affichage (1, 2) soit branchable à une mémoire de textes d'un système d'information électronique (3) par un placement de commutateurs (S1-b) destiné à l'indication d'information qui peut être appelée de la mémoire de textes sur le dispositif d'affichage (1, 2) par un commutateur d'appel (3-S3).

8. Installation radiographique selon l'une des revendications 1 à 7, caractérisé en ce que le dispositif d'affichage (1, 2) et un raccordement de clavier (6) soient branchés à une mémoire imprimeuse (13) qui peut être extraite par un commutateur d'appel (13-S3) dans une imprimante, surtout une imprimeuse d'étiquettes.

9. Installation radiographique selon l'une des revendication 1 à 8, caractérisé en ce que l'électronique de contrôle integré et programmé (8) ait une mémoire de texte de contrôle qui est branchable au dispositif d'affichage alphanumérique à plusieurs chiffres (1, 2) par un placement de commutateurs (S2-b) et par un commutateur de sélection (8-S3).

10. Installation radiographique selon l'une des revendications 1 à 9, caractérisé en ce qu'il y ait un générateur radiographique et plusieurs appareils radiographiques, qui sont facultativement branchables du pupitre de commande au dispositif d'affichage en actionnant le commutateur respectivement coordoné, sont branchés par une matrice de circuit combinatoire (7), qui peut être commutée par commande par programme et par un placement de commutateurs (S2-c) en actionnant des commutateurs de commande (7-S3, 7-S4, 7S5) et à laquelle une mémoire de textes pour matrices de circuits combinatoires est coordonnée, qui peut être appelée sur le dispositif d'affichage alphanumérique à plusieurs chiffres (1, 2) par le placement de commutateurs (S2-c) en actionnant le commutateur de commande.